# EUROPEAN PATENT APPLICATION

(11) **EP 4 796 522 A1**
(43) Date of publication of application: **26.08.2026**
(21) Application number: 24907655.5
(22) Date of filing: 29.07.2024
(51) Int. Cl.: C07C 1/20, C07C 29/151, C07C 11/04, C07C 11/06, C07C 31/04, C01B 3/40, C01B 3/48, C10J 3/84, C10G 1/10

(54) **METHOD AND APPARATUS FOR CONVERTING WASTE PLASTICS INTO LIGHT OLEFINS AT HIGH YIELD**

(30) Priority: 18.12.2023 KR 20230184233
(71) Applicant: SK Innovation Co., Ltd., Seoul 03188 (KR)
(72) Inventor: JEON, Heejung, Daejeon 34124 (KR); KIM, Okyoun, Daejeon 34124 (KR); YUN, Dongmin, Daejeon 34124 (KR)
(74) Representative: Lorenz Seidler Gossel Part. mbB
(86) International application number: PCT/KR2024/011006
(87) International publication number: WO 2025/135368

(57) **Abstract**

The present disclosure relates to a method for producing light olefins and, more particularly, to a method for producing light olefins which can increase the production yield of light olefins and minimize the generation of carbon dioxide. The method for producing light olefins according to the present disclosure comprises the steps of: S1) pyrolyzing waste plastics to produce pyrolysis oil and pyrolysis gas; S2) separating the pyrolysis oil and pyrolysis gas; S3) producing a first gas from which impurities are removed by purifying the separated pyrolysis gas; S4) steam-reforming the first gas to manufacture a second gas; S5) producing a first synthesis gas from the second gas; S6) producing a second synthesis gas by converting carbon monoxide in the synthesis gas into hydrogen and carbon dioxide through a water gas shift reaction; S7) preparing a first mixed solution containing methanol by converting the second synthesis gas into methanol through a hydrogenation reaction; and (S8) preparing a second mixed solution containing light olefins through an olefin conversion reaction of methanol contained in the first mixed solution, and recovering the light olefins from the second mixed solution.

## Description

### [Technical Field]

The present disclosure relates to a method and apparatus for producing light olefins from waste plastics used as a feedstock, and more particularly, to a method and apparatus for producing light olefins that may increase a production yield of the light olefins from the waste plastics and minimize generation of carbon dioxide.

### [Background Art]

Waste plastics, which are produced using petroleum as a feedstock, have low recyclability and are mostly disposed of as waste. Such wastes require a long time to decompose in a natural state, thereby contaminating soil and causing serious environmental pollution. As a method for recycling waste plastics, there is a method of pyrolyzing waste plastics to convert the waste plastics into usable oil.

Since waste plastics are a mixture of hydrocarbon fractions having various boiling points and various molecular weight distributions, in a process of producing pyrolysis oil from the waste plastics, liquid pyrolysis oil and pyrolysis gas generated in a gaseous phase are produced simultaneously, and the amount of the pyrolysis gas is approximately the same as that of the liquid pyrolysis oil. The liquid pyrolysis oil is used as fuel and as a feedstock for various petrochemical industries after undergoing separation and refining processes, whereas the pyrolysis gas is reused as fuel for pyrolysis or discharged into the atmosphere after passing through a heat exchanger. However, since such exhaust gas contains a large amount of carbon dioxide, concerns have been raised that greenhouse gas emission standards must be satisfied, and other impurities contained in the exhaust gas may cause environmental pollution.

Meanwhile, light olefins refer to ethylene, propylene, and butene obtained from naphtha cracking and are essential basic feedstocks in the petrochemical industry for producing various chemical products such as synthetic resins, synthetic rubber, and alcohols. To date, most ethylene or propylene has been produced by pyrolysis of natural gas or hydrocarbon fractions mainly composed of paraffinic compounds, such as naphtha fractions and gas oil, under non-catalytic conditions in a steam atmosphere at a high temperature of 800°C or higher. However, in recent years, due to a rapid increase in crude oil prices, prices of feedstocks for light olefins have increased significantly, and a large amount of carbon dioxide, which causes global warming, is emitted in a process of producing light olefins. Thus, there is a need to develop a process for producing light olefins from resources other than crude oil in order to reduce such carbon dioxide emissions.

In order to solve the above-described problems, attempts have been made to produce such light olefins from waste plastic pyrolysis oil. However, the waste plastic pyrolysis oil contains an excessive amount of impurities such as chlorine, nitrogen, or metals as compared with fuel components, such that reaction activity is significantly reduced and conversion efficiency is significantly low. In addition, it is difficult to produce high-quality light olefins, and thus it is difficult to commercially and economically put this approach into practice. Pyrolysis gas generated during pyrolysis of waste plastics contains a lower amount of impurities such as chlorine and nitrogen than pyrolysis oil while containing a large amount of hydrocarbons such as methane and olefins, and thus has attracted attention as a feedstock for light olefins. However, a process of producing light olefins from pyrolysis gas has low productivity, and there have been difficulties in practical application and commercialization.

Accordingly, there is a need for research on a method and apparatus capable of converting waste plastics into light olefins at a high yield.

### [Related Art Document]

### [Patent Document]

Korean Patent Laid-Open Publication No. 10-2023-0002685 A (2023.01.05)

### [Disclosure]

### [Technical Problem]

According to an aspect of the present disclosure, it is possible to provide a method and apparatus capable of converting waste plastics into light olefins at a high yield through pyrolysis of the waste plastics.

According to another aspect of the present disclosure, it is possible to provide a method and apparatus for producing light olefins that may minimize generation of carbon dioxide and may implement carbon neutrality.

### [Technical Solution]

In one general aspect, a method for producing light olefins includes: S1) pyrolyzing waste plastics to produce pyrolysis oil and pyrolysis gas; S2) separating the pyrolysis oil and the pyrolysis gas; S3) purifying the separated pyrolysis gas to produce a first gas from which impurities are removed; S4) subjecting the first gas to a steam reforming reaction to produce a second gas; S5) producing a first syngas from the second gas; S6) converting carbon monoxide in the first syngas into hydrogen and carbon dioxide through a water-gas shift reaction to produce a second syngas; S7) converting the second syngas into methanol through a methanol conversion reaction; and S8) converting the methanol into light olefins through an olefin conversion reaction.

In an embodiment, the step S3) may include: removing impurities from the separated pyrolysis gas to produce the first gas; and separating olefins from the first gas.

In an embodiment, the olefins may be mixed with the light olefins in the step S8) and recovered.

In an embodiment, the impurities may include one or two or more selected from the group consisting of tar, sulfur, nitrogen, and chlorine.

In an embodiment, in the step S4), the second gas may contain hydrogen, carbon monoxide, and carbon dioxide.

In an embodiment, the step S5) may include: separating, from the second gas, a first stream containing carbon dioxide and a second stream containing hydrogen and carbon monoxide; converting the first stream into a third stream containing carbon monoxide through a reverse Boudouard reaction; and mixing the third stream and the second stream to produce the first syngas.

In an embodiment, the pyrolysis oil may contain one or more fractions selected from the group consisting of an aromatic fraction, naphtha, and a heavy fraction.

In an embodiment, a pyrolysis temperature in the step S1) may be 400°C to 600°C.

In an embodiment, in the step S4), the steam reforming reaction may be performed in the presence of a catalyst, and the catalyst may be a composite catalyst in which a hydrogenation metal is supported on a support.

In an embodiment, the hydrogenation metal may be one or more selected from the group consisting of nickel, vanadium, iron, platinum, palladium, and ruthenium.

In an embodiment, the reverse Boudouard reaction may be performed at a temperature of 800°C to 1,000°C and a pressure of 50 KPa to 200 KPa.

In an embodiment, the second syngas may contain hydrogen and carbon monoxide, and a molar ratio of hydrogen to carbon monoxide may be 1.9 to 2.1:1.

In an embodiment, the step S7) may be performed at a temperature of 400°C to 600°C and a pressure of 1 bar to 10 bar.

In an embodiment, the methanol conversion product in the step S7) may contain methanol in an amount of 10 wt% or more based on the total weight of the product.

In an embodiment, the step S8) may be performed in the presence of a zeolite-based catalyst or an AlPO₄-based molecular sieve catalyst.

In an embodiment, the zeolite-based catalyst or the AlPO₄-based molecular sieve catalyst may be ZSM-5, SAPO-34, or a combination thereof.

In another general aspect, there is provided an apparatus for producing light olefins.

The apparatus for producing light olefins according to the present disclosure includes: a first reactor configured to perform pyrolysis of organic waste to produce pyrolysis oil and pyrolysis gas; a purification unit configured to receive the pyrolysis gas and produce purified pyrolysis gas; a second reactor configured to receive the purified pyrolysis gas and produce a second gas through a steam reforming reaction; a first syngas production unit configured to produce a first syngas from the second gas; a second syngas production unit configured to convert carbon monoxide in the first syngas into hydrogen and carbon dioxide through a water-gas shift reaction to produce a second syngas; a third reactor configured to produce methanol from the second syngas through a methanol conversion reaction; and a fourth reactor configured to produce light olefins from the methanol through an olefin conversion reaction.

In an embodiment, the purification unit may include an impurity removal unit configured to remove impurities; and an olefin separation unit configured to separate and recover olefins.

In an embodiment, the first syngas production unit may include: an amine scrubber configured to receive the second gas and separate carbon dioxide; and a reverse Boudouard reactor configured to receive the carbon dioxide separated from the amine scrubber and perform a reverse Boudouard reaction.

In an embodiment, the first to fourth reactors may include a fluidized bed reactor or a fixed bed reactor.

In an embodiment, the reverse Boudouard reactor may include a fluidized bed reactor or a fixed bed reactor.

### [Advantageous Effects]

According to an embodiment of the present disclosure, light olefins may be produced with high efficiency by pyrolyzing waste plastics.

According to an embodiment of the present disclosure, generation of carbon dioxide may be minimized in a process of producing light olefins.

### [Description of Drawings]

FIG. 1 is a schematic diagram illustrating a method for producing light olefins according to the present disclosure.
FIG. 2 is a schematic diagram illustrating a method for producing light olefins according to an embodiment of the present disclosure.
FIG. 3 is a schematic diagram illustrating an apparatus for producing light olefins according to the present disclosure.
FIG. 4 is a schematic diagram illustrating an apparatus for producing light olefins according to an embodiment of the present disclosure.
FIG. 5 is a schematic diagram illustrating an olefin separation unit according to an embodiment of the present disclosure.

### [Best Mode]

Unless the context clearly indicates otherwise, singular forms used in the present specification may be construed to include plural forms.

A numerical range used in the present specification includes upper and lower limits and all values therebetween, all values defined by double limitations, and all possible combinations of upper and lower limits of numerical ranges defined in different forms. Unless otherwise specifically defined in the present specification, values outside the numerical range that may occur due to experimental errors or rounding also fall within the defined numerical range.

The term "comprise(s)" described in the present specification is an open-ended description having a meaning equivalent to the term such as "include(s)", "contain(s)", "have(has)", or "is/are characterized by", and does not exclude elements, materials, or processes which are not further listed.

Unless otherwise defined, the unit of % used in the present specification without special mention refers to wt%.

Since waste plastics are a mixture of hydrocarbon fractions having various boiling points and various molecular weight distributions, in a process of producing pyrolysis oil from the waste plastics, liquid pyrolysis oil and pyrolysis gas generated in a gaseous phase are produced simultaneously, and the amount of the pyrolysis gas is as much as that of the liquid pyrolysis oil. Such pyrolysis gas is mostly discharged as exhaust gas even though the pyrolysis gas contains hydrocarbons that serve as a feedstock for light olefins. Accordingly, the inventors of the present disclosure devised a method and apparatus capable of producing light olefins with high efficiency by pyrolyzing waste plastics.

The present disclosure provides a method for producing light olefins, the method including: S1) pyrolyzing waste plastics to produce pyrolysis oil and pyrolysis gas; S2) separating the pyrolysis oil and the pyrolysis gas; S3) purifying the separated pyrolysis gas to produce a first gas; S4) subjecting the first gas to a steam reforming reaction to produce a second gas; S5) producing a first syngas from the second gas; S6) converting carbon monoxide in the first syngas into hydrogen and carbon dioxide through a water-gas shift reaction to produce a second syngas; S7) converting the second syngas into methanol through a methanol conversion reaction; and S8) converting the methanol into light olefins through an olefin conversion reaction.

The method for producing light olefins according to the present disclosure may minimize generation of carbon dioxide and thus may prevent environmental pollution as compared with a conventional process for producing light olefins; may recover liquid pyrolysis oil in a process of pyrolyzing waste plastics; and may also recover high value-added light olefins through pyrolysis of the waste plastics, thereby efficiently converting waste resources.

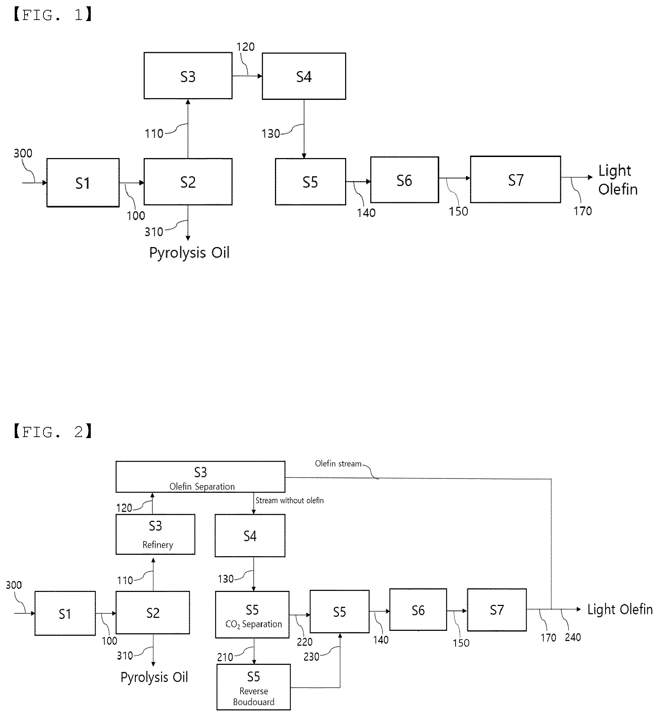

The step S1) is a step of pyrolyzing waste plastics to produce pyrolysis oil and pyrolysis gas. Specifically, referring to FIG. 1, waste plastics 300 are introduced into a pyrolysis reactor, pyrolysis is performed, and pyrolysis gas and pyrolysis oil 100 are produced.

Specifically, the waste plastics 300 may be household plastic waste (household waste plastics) or industrial plastic waste (industrial waste plastics). The household waste plastics may be polyolefin-based waste plastics, and may be, specifically, plastics in which PVC, PS, PET, and PBT, in addition to PE and PP, are mixed.

The pyrolysis reaction may be performed in a batch reactor. Specifically, the pyrolysis reaction may be performed in any reactor capable of controlling stirring and temperature increase, and, for example, pyrolysis may be performed in a rotary kiln-type batch reactor; however, the present disclosure is not limited thereto.

The pyrolysis temperature may be, specifically, 300°C to 900°C, and more specifically, 400°C to 600°C.

The pyrolysis reaction may be performed in a non-oxidizing atmosphere. The non-oxidizing atmosphere is an atmosphere in which waste plastics do not oxidize (burn), and efficient pyrolysis may be performed in the atmosphere. The non-oxidizing atmosphere may be, for example, an atmosphere in which an oxygen concentration is adjusted to 1 vol% or less, and may be an atmosphere of an inert gas such as nitrogen, steam, carbon dioxide, or argon. The pyrolysis process may be stably performed in a low-oxygen atmosphere in which an oxygen concentration is adjusted to 1 vol% or less. The pyrolysis reaction may be performed under a non-oxidizing atmosphere for 50 minutes to 550 minutes, specifically, 150 minutes to 350 minutes, and, when the holding time is satisfied, activation of the non-oxidizing atmosphere composition and sufficient pyrolysis may be achieved, and energy consumption and operating time may be minimized, which is preferable.

The step S2) is a step of gas-liquid separating the pyrolysis oil and the pyrolysis gas 100 to recover pyrolysis oil 310 and collect only pyrolysis gas 110.

Referring to FIG. 1, the pyrolysis oil and the pyrolysis gas 100 produced by pyrolyzing waste plastics are gas-liquid separated, such that the pyrolysis oil 310 in a liquid phase may be recovered, and only the pyrolysis gas 110 in a gaseous phase may be collected and introduced into a subsequent process.

The pyrolysis gas 110 may contain hydrogen, carbon monoxide, carbon dioxide, methane, olefins, and C2 to C4 hydrocarbons.

The pyrolysis oil 310 may contain one or more fractions selected from the group consisting of an aromatic fraction, naphtha, and a heavy fraction.

According to an embodiment, the pyrolysis oil 310 recovered in the step S2) may be recovered as a specific fraction through refining and separation processes.

The step S3) is a step of purifying the pyrolysis gas 110 separated in the step S2) to produce a first gas 120.

The pyrolysis gas 110 produced by pyrolyzing waste plastics may contain one or two or more impurities selected from the group consisting of tar, sulfur, nitrogen, and chlorine. Specifically, the pyrolysis gas 110 may contain water-soluble impurities such as H₂S, HCl, HOCl, and NH₃, and water-insoluble impurities such as tar. Such impurities contained in the pyrolysis gas 110 may induce deactivation of the catalyst and thus may reduce efficiency of a subsequent process. Accordingly, when impurities are removed from the pyrolysis gas 110 to purify the pyrolysis gas 110, efficiency of the overall process may be improved.

A method of purifying the pyrolysis gas 110 may be one or a combination of two or more selected from the group consisting of use of a high-pressure dust collection filter, washing with water, washing with an alkaline solution, and passage through a ceramic filter. However, the method is not limited to the above methods as long as impurities contained in the pyrolysis gas 110 may be removed. When washing with water or washing with an alkaline solution is used, water-soluble impurities such as H₂S, HCl, HOCl, and NH₃ contained in the pyrolysis gas 110 may be removed, and when the pyrolysis gas is passed through a ceramic filter or a dust collection filter, water-insoluble impurities such as tar and dust may be removed. Through the above-described purification process, the pyrolysis gas 110 may be purified and converted into the first gas 120.

The first gas 120 may contain hydrogen, carbon monoxide, carbon dioxide, methane, olefins, and C2 to C4 hydrocarbons.

In an embodiment of the present disclosure, referring to FIG. 2, the step S3) may include: removing impurities from the separated pyrolysis gas 110 to produce the first gas 120; and separating olefins from the first gas 120.

The step of removing impurities from the pyrolysis gas 110 may be the same as the above-described impurity removal method. In the first gas 120, olefins contained in the first gas 120 may be separated by an olefin separation process to form an olefin stream, and components other than the olefins contained in the first gas 120 may be introduced into the step S4), which is a subsequent process.

Since the step S3) includes an impurity removal process and an olefin separation process, the olefin stream separated from the pyrolysis gas 110 may be mixed and recovered together with an olefin-containing product 170 finally converted from the pyrolysis gas 110, thereby further improving the overall olefin recovery. In addition, since there is no need to additionally convert the olefins into hydrogen and carbon monoxide in a subsequent steam reforming process, more efficient process operation may be achieved.

The step of separating the olefins may be performed by passing the first gas 120 through an adsorption column packed with an adsorbent and a distillation column, and may include: supplying the first gas 120 to the adsorption column to adsorb olefins; and supplying the adsorbed olefins to the distillation column to recover olefins.

As a non-limiting example, the first gas 120 containing olefins may be transferred to the adsorption column packed with the adsorbent and come into contact with the adsorbent. In this case, the olefins contained in the first gas 120 are adsorbed on the adsorbent to form an olefin adsorbate, such that the olefins contained in the first gas 120 are separated from the first gas 120, and the olefin adsorbate may be transferred to the distillation column. In the distillation column, the olefin adsorbate may be separated into an olefin stream and an adsorbent by distillation. The separated olefin stream may be mixed and recovered together with the olefin-containing product 170 finally converted from the pyrolysis gas, and the adsorbent may be reintroduced into the olefin separation step and reused.

The adsorbent packed in the adsorption column may include one or more selected from the group consisting of a π-complexation adsorbent that selectively forms an adsorbate with olefins, an X-type zeolite adsorbent, a Y-type zeolite adsorbent, and an A-type zeolite adsorbent.

The step of adsorbing the olefins may be performed under conditions of a pressure of 1 atm to 35 atm and a temperature of 20°C to 150°C.

The step of recovering the olefins may be performed under conditions of a pressure of 1 atm to 35 atm and a temperature of 20°C to 150°C.

Since the step S3) further includes a purification process and an olefin separation process, a gas supplied to the step S4) may contain hydrogen, carbon monoxide, carbon dioxide, methane, and C2 to C4 hydrocarbons.

The step S4) is a step of converting a product of the step S3) into a second gas 130 through a steam reforming reaction. In the step S4), hydrocarbons contained in the product of the step S3) may be reacted with steam through a steam reforming reaction to be converted into carbon monoxide and hydrogen gas, thereby producing the second gas 130. In the steam reforming reaction, one or two or more of the reforming reactions among the following reaction schemes may be performed.

[Reaction Scheme 1] CH₄ + H₂O → CO + 3H₂

[Reaction Scheme 2] CₓH_{y} + nH₂O→ xCO + (x + y/2)H₂

The second gas 130 may contain hydrogen and carbon monoxide, and may further contain carbon dioxide and methane unreacted in the steam reforming reaction.

The steam reforming reaction in the step S4) may be performed at a temperature of 600°C to 1,400°C and a pressure of 30 KPa to 2,000 KPa.

The step S4) may be operated under non-catalytic conditions, but may be operated using a catalyst to increase a reaction conversion at a low temperature of 600°C to 700°C. As the catalyst in the step S4), a composite catalyst in which a hydrogenation metal is supported on a support may be used. The hydrogenation metal may include one or two or more selected from nickel, vanadium, iron, platinum, palladium, and ruthenium. The hydrogenation metal may be a commonly known metal such as nickel, vanadium, or iron, and a noble metal may be used when a content of impurities in a feedstock such as organic waste is low in a heat treatment process. The noble metal may be platinum, palladium, or ruthenium. As the support, a solid acid material such as an oxide or a zeolite may be used. Specifically, as the support, ZSM-5, ZSM-11, USY zeolite, ferrierite, mordenite, MCM-22, SUZ-4, or L-type zeolite, silica, alumina, silica-alumina, carbon, zirconia, titania, and the like may be used.

Referring to FIG. 1, the step S5) is a step of producing a first syngas 140 from the second gas 130 produced in the step S4).

The first syngas 140 may contain hydrogen and carbon monoxide as main components.

In an embodiment, referring to FIG. 2, the step S5) may include: separating, from the second gas 130, a first stream 210 containing carbon dioxide and a second stream 220 containing hydrogen and carbon monoxide; converting the first stream 210 into a third stream 230 containing carbon monoxide through a reverse Boudouard reaction; and mixing the third stream 230 and the second stream 220 to produce the first syngas 140.

Referring to FIG. 2, the second gas 130 may be introduced into a carbon dioxide separation process and separated into the first stream 210 containing carbon dioxide and the second stream 220 containing hydrogen and carbon monoxide, such that the first stream 210 may be supplied to a subsequent step for performing a reverse Boudouard reaction and the second stream 220 may be supplied to a first syngas production unit. The first stream 210 may be converted into carbon monoxide through a reverse Boudouard reaction to form the third stream 230, and may be introduced into the first syngas production unit. In the first syngas production unit, the second stream 220 and the third stream 230 may be mixed to produce the first syngas 140. Since the step S5) includes the above-described steps according to an embodiment, a content of carbon monoxide, which is a reactant in a subsequent water-gas shift reaction, may be increased and a content of carbon dioxide, which is a product of the reaction, may be decreased. Thus, reactivity of the water-gas shift reaction may be improved, and as a result, efficiency of the overall process for producing light olefins may be improved.

The step of separating the carbon dioxide from the second gas 130 is not limited as long as the step is capable of separating carbon dioxide from the second gas 130, and may be performed using various carbon dioxide separation units. In an embodiment, the separation may be performed using an amine scrubber. In general, an amine scrubber, which binds and removes carbon dioxide using an amine-based material, may separate components such as carbon dioxide and hydrogen sulfide from a gas stream and recover a gas containing hydrogen, carbon monoxide, or an inert gas.

Specifically, in a first amine scrubber step, the second gas may be introduced into an amine solution at a temperature of 40°C to 100°C to capture carbon dioxide, and, in a second amine scrubber step, the amine solution may be heated to 100°C to 200°C to be separated into the amine solution and carbon dioxide. When the amine scrubber as described above is used, carbon dioxide may be separated at a lower temperature as compared with a CCS unit described below, which may be advantageous in terms of process stability.

In another embodiment, the separation of carbon dioxide may be performed using a carbon capture and storage (CCS) unit. The CCS unit may adsorb and separate carbon dioxide using an adsorbent including one or two or more selected from calcium oxide, calcium hydroxide, dolomite, limestone, and trona. Specifically, the adsorbent may be calcium oxide. In the CCS unit, adsorption of the carbon dioxide may be performed at a temperature of 500°C or higher and lower than 800°C and a pressure of 300 KPa to 500 KPa. Under the above conditions, carbon dioxide adsorption efficiency may be excellent. Specifically, the temperature may be 500°C to 700°C and the pressure may be 150 KPa to 500 KPa, and more specifically, the temperature may be 550°C to 650°C and the pressure may be 50 KPa to 100 KPa. In the CCS unit, the carbon dioxide adsorbed on the adsorbent may be desorbed again, and the desorption may be performed at a temperature of 500°C to 1,000°C and a pressure of 300 KPa to 500 KPa. Under the above conditions, carbon dioxide desorption efficiency may be excellent. Specifically, the temperature may be 700°C to 950°C and the pressure may be 50 KPa to 150 KPa, and more specifically, the temperature may be 850°C to 950°C and the pressure may be 50 KPa to 100 KPa.

The step of converting the first stream 210 into the third stream 230 containing carbon monoxide through the reverse Boudouard reaction is a step of converting carbon dioxide contained in the first stream 210 into carbon monoxide through a reverse Boudouard reaction. By additionally converting carbon dioxide into carbon monoxide through a reverse Boudouard reaction, environmental pollution may be prevented in terms of reducing carbon dioxide emissions. In addition, in terms of increasing a content of reactants in a subsequent process, the overall production yield of light olefins may be improved. The reverse Boudouard reaction may involve the following Reaction Scheme 3.

[Reaction Scheme 3] C + CO₂ → 2CO

In an embodiment, in order to smoothly perform the reverse Boudouard reaction, the reverse Boudouard reaction may be performed by supplying carbon such as activated carbon, and, in another embodiment, a catalyst deactivated by coke in the step S1) may be supplied and used as a carbon source to perform the reverse Boudouard reaction. Specifically, the carbon source may be char derived from the organic waste, and more specifically, may be char derived from pyrolysis of waste plastics or char derived from biomass. Alternatively, high-purity graphite may be included to prevent entry of impurity gases due to an external carbon source.

When unreacted carbon dioxide remains during the reverse Boudouard reaction, carbon dioxide may be separated again from the product, and the reverse Boudouard reaction may be repeatedly performed one or more times on the separated carbon dioxide. Accordingly, carbon dioxide may be converted into carbon monoxide with remarkably high efficiency.

The reverse Boudouard reaction may be performed at a temperature of 700°C to 1,000°C and a pressure of 50 KPa to 200 KPa. Under the above conditions, conversion efficiency of carbon dioxide into carbon monoxide and catalyst regeneration efficiency may be excellent. Specifically, the temperature may be 800°C to 1,000°C and the pressure may be 50 KPa to 150 KPa, and more specifically, the temperature may be 950°C to 1,000°C and the pressure may be 100 KPa to 150 KPa.

The step of producing the first syngas 140 may refer to mixing the second stream 220 separated from the second gas 130 with the third stream 230 produced by converting the first stream 210 through a reverse Boudouard reaction, and the two streams may be mixed to produce the first syngas 140.

The step S6) is a step of producing a second syngas 150 by converting carbon monoxide contained in the first syngas 140 produced in the step S5) into hydrogen through a water-gas shift reaction. The water-gas shift reaction may involve the following Chemical Formula 4.

[Chemical Formula 4] CO + H₂O → H₂ + CO₂

The water-gas shift reaction may be performed in the presence of a catalyst including one or more metals selected from the group consisting of Fe, Cr, Cu, and Zn. The water-gas shift reaction may be performed at a temperature of 100°C to 400°C, specifically 100°C to 300°C, and a pressure of 20 bar to 80 bar, specifically 25 bar to 70 bar.

In the second syngas produced by the water-gas shift reaction, a ratio of hydrogen to carbon monoxide may be 1.5 to 3:1, specifically 1.9 to 2.1:1. When the ratio of hydrogen to carbon monoxide in the second syngas satisfies the above-described range, a subsequent methanol synthesis process may be efficiently performed.

The step S7) is a step of producing methanol through a methanol conversion reaction using hydrogen and carbon monoxide contained in the second syngas 150 as reactants. A hydrogenation reaction of carbon monoxide may involve the following Chemical Formula 5.

[Chemical Formula 5] CO + 2H₂ → CH₃OH

In an embodiment, the step S7) may be performed in the presence of a Cu-based catalyst. Specifically, in order to facilitate methanol conversion at a low temperature, the Cu-based catalyst may be a Cu-based methanol synthesis catalyst, and one or more selected from the group consisting of SiO₂, ZrO₂, Ga₂O₃, Al₂O₃, MgO, and TiO₂ may be used as a support of the Cu-based catalyst. Specifically, the Cu-based methanol synthesis catalyst may be Cu/Zr/Al₂O₃.

The step S7) may be performed at 400°C to 600°C, specifically 430°C to 530°C, and 0.1 MPa to 10 MPa, specifically 0.1 MPa to 5 MPa.

Referring to FIG. 2, the second syngas 150 may produce a methanol-containing product 160 containing methanol and water through a methanol conversion reaction. The product may contain methanol in an amount of 10 wt% or more, 20 wt% or more, 30 wt% or more, 50 wt% or more, 90 wt% or less, 80 wt% or less, or 70 wt% or less, specifically 10 to 90 wt%, and more specifically 30 to 70 wt%, based on the total content thereof.

Methanol contained in the methanol-containing product 160 may be converted into an olefin-containing product 170 through an olefin conversion reaction. Methanol contained in the methanol-containing product 160 of the step S7) may be converted into olefins through contact with an olefin conversion catalyst in the step S8).

In an embodiment, the step S8) may be performed in the presence of a zeolite-based catalyst or an AlPO₄-based molecular sieve catalyst. Specifically, the zeolite-based catalyst may include ZSM-5, and the AlPO₄-based catalyst may be a silicoaluminophosphate (SAPO) molecular sieve catalyst, and may be, specifically, one or more selected from SAPO-5, SAPO-8, SAPO-11, SAPO-16, SAPO-17, SAPO-18, SAPO-20, SAPO-31, SAPO-34, SAPO-35, SAPO-44, and SAPO-46.

The step S8) may be performed at a temperature of 200°C to 600°C, specifically 300°C to 500°C, and a pressure of 1 bar to 10 bar, specifically 1 bar to 5 bar.

In an embodiment, the step S8) may further include removing moisture from the olefin-containing product 170. Since the olefin-containing product 170 may contain water and light olefins, the step S8) may further include removing moisture to recover only olefins. A method of removing moisture may use a known method, and is not limited thereto.

In an embodiment, the olefin-containing product 170 recovered in the step S8) may be mixed with the olefin stream separated in the step S3) and recovered as an olefin product 240, and moisture may be removed from the olefin product 240 to recover only olefins.

The present disclosure provides an apparatus 1000 for producing light olefins, the apparatus including: a first reactor 10 configured to perform pyrolysis of organic waste to produce pyrolysis oil and pyrolysis gas; a purification unit 20 configured to receive the pyrolysis gas and produce purified pyrolysis gas; a second reactor 30 configured to receive the purified pyrolysis gas and produce a second gas through a steam reforming reaction; a first syngas production unit 40 configured to receive the second gas from the second reactor 30 and produce a first syngas 140; a second syngas production unit 50 configured to receive the first syngas 140 and convert carbon monoxide into hydrogen and carbon dioxide through a water-gas shift reaction to produce a second syngas 150; a third reactor 60 configured to receive the second syngas 150 and produce methanol through a methanol conversion reaction; and a fourth reactor 70 configured to produce light olefins from the methanol through an olefin conversion reaction.

By using the apparatus for producing light olefins according to the present disclosure, light olefins may be produced from waste plastics at a high yield through pyrolysis of the waste plastics. In addition, by capturing carbon dioxide discharged as exhaust gas and using the captured carbon dioxide as a feedstock for producing light olefins, environmental pollution may be prevented. In addition, when pyrolysis oil produced during pyrolysis is connected to a post-treatment process of the pyrolysis oil, high-quality pyrolysis oil may also be recovered. That is, since waste plastics are pyrolyzed to convert not only pyrolysis oil but also pyrolysis gas produced during pyrolysis to obtain high value-added hydrocarbons together, an effect of recycling waste resources may be excellent.

In an embodiment, the first and fourth reactors used in the apparatus for producing light olefins according to the present disclosure may be a batch reactor, and may be a fluidized bed reactor or a fixed bed reactor. Each reactor may be a fluidized bed reactor or a fixed bed reactor without limitation; however, when a fluidized bed reactor is used, it may be advantageous in terms of process efficiency.

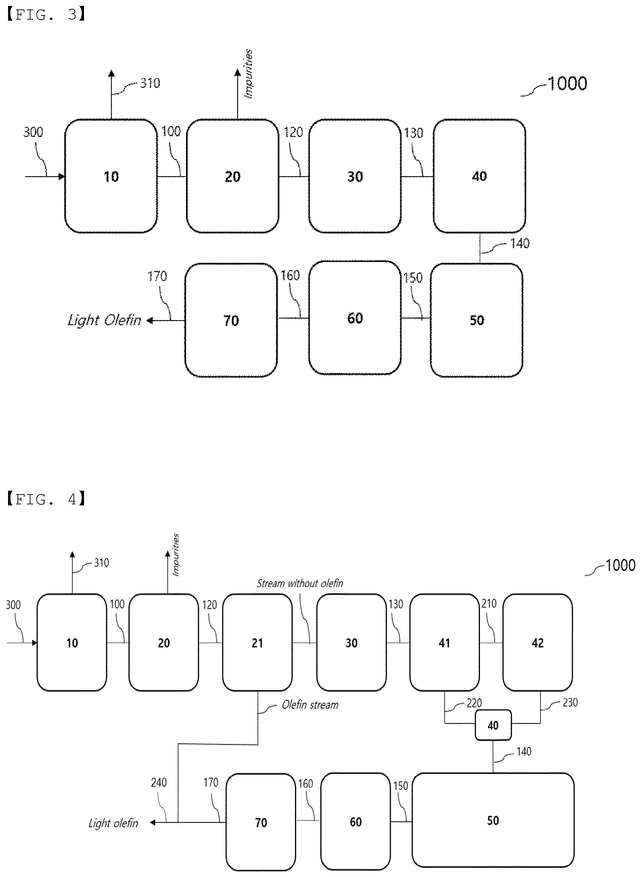

Referring to FIG. 3, waste plastics 300 are introduced into the first reactor 10, and a pyrolysis reaction is performed so that the waste plastics 300 are converted into pyrolysis oil 310 and pyrolysis gas 100, which are liquid-phase and gas-phase hydrocarbons having various boiling points. The pyrolysis oil 310 is transferred to a post-treatment process along a pipe connected to the first reactor for post-treatment, and the pyrolysis gas 100 is supplied to the purification unit 20.

The purification unit 20 may include a sprayer for spraying a liquid to bring the pyrolysis gas 100 into contact with water or a weakly basic solution containing sodium carbonate to remove water-soluble impurities such as H2S, HCl, HOCl, and NH3; may remove dust using a high-pressure dust collection filter; and may include a ceramic filter to remove water-insoluble impurities such as tar.

In an embodiment, the purification unit 20 may include: an impurity removal unit 20 configured to remove impurities; and an olefin separation unit 21 configured to separate and recover olefins.

Referring to FIG. 4, the pyrolysis gas 100 produced in the first reactor is introduced into the purification unit 20 to discharge impurities to the outside, and a first gas 120 from which impurities are removed is introduced into the olefin separation unit 21. By the olefin separation unit 21, olefins contained in the first gas 120 are separated to form an olefin stream, and the first gas from which olefins are separated is transferred to the second reactor 30. In this case, the olefin stream separated from the first gas may be mixed and recovered together with an olefin-containing product 170 finally converted from the pyrolysis gas.

Referring to FIGS. 3 and 4, the second reactor 30 receives the first gas 120 from the purification unit 20 and converts the first gas 120 into a second gas 130 through a steam reforming reaction. Specifically, in the second reactor 30, the first gas 120 supplied from the purification unit 20 may be converted into hydrogen, carbon monoxide, and carbon dioxide through a steam reforming reaction of hydrocarbons contained in the first gas 120, thereby producing the second gas 130 containing hydrogen and carbon monoxide as main components.

Referring to FIG. 3, the first syngas production unit 40 receives the second gas 130 from the second reactor 30 and produces the first syngas 140.

In an embodiment, referring to FIG. 4, the first syngas production unit 40 may further include: an amine scrubber 41 configured to receive the second gas 130 and separate carbon dioxide; and a reverse Boudouard reactor 42 configured to receive the carbon dioxide separated from the amine scrubber 41 and perform a reverse Boudouard reaction.

Since the first syngas production unit 40 further includes the amine scrubber 41 and the reverse Boudouard reactor 42, carbon dioxide contained in the first syngas may be converted into carbon monoxide, thereby reducing carbon dioxide emissions to prevent environmental pollution, and improving reactivity of a subsequent methanol conversion process.

Referring to FIG. 4, the second gas 130 produced in the second reactor 30 may be introduced into the amine scrubber 41 and separated into a first stream 210 containing carbon dioxide and a second stream 220 not containing carbon dioxide. The first stream 210 may be introduced into the reverse Boudouard reactor 42 to convert carbon dioxide contained in the first stream 210 into carbon monoxide, thereby producing a third stream 230 containing carbon monoxide, and the third stream 230 may be mixed with the second stream 220 separated in the amine scrubber 41 and may be supplied to the first syngas production unit 40 as the first syngas 140.

The second syngas production unit 50 receives the first syngas 140 from the first syngas production unit 40, and carbon monoxide contained in the first syngas 140 reacts with steam to be converted into hydrogen, thereby producing the second syngas 150.

The second syngas 150 may contain hydrogen and carbon monoxide as main components.

The third reactor 60 may receive the second syngas 150 containing carbon monoxide and hydrogen from the second syngas production unit 50, convert hydrogen and carbon monoxide into methanol through a methanol conversion reaction, and produce a product containing the methanol.

The fourth reactor 70 receives a product containing methanol from the third reactor 60 and converts methanol in a first mixed liquid into light olefins, thereby producing a product containing olefins and water.

In an embodiment, a second mixed liquid produced in the fourth reactor 70 may be supplied to a separation column to separate water and the olefin-containing product 170, such that only the olefin product 170 may be recovered, and the separated olefins may be mixed and recovered together with the olefin stream separated in the olefin separation unit 21.

Hereinafter, the present disclosure will be described in more detail with reference to examples.

### (Example 1)

A waste plastic mixture was dried in an oven at 60°C to remove moisture in the feedstock. The composition of the dried waste plastic mixture is shown in Table 1.

**[Table 1]**

| | PET | PE | PP | PS | PC | PVC | Others |
|---|---|---|---|---|---|---|---|
| Composition (%) | 3.7 | 60.3 | 23.8 | 0.4 | 2.8 | 0.1 | 9.0 |

5,000 g of the dried waste plastic mixture was introduced into a first reactor of a rotary kiln type, pyrolysis was performed by treating the mixture at 600°C for 1 hour, and then pyrolysis gas and pyrolysis oil were separated. The yields of the pyrolysis oil and the pyrolysis gas included in the recovered pyrolysis products are as shown in Table 2, and the composition of the pyrolysis gas is as shown in Table 3.

**[Table 2]**

| Recovered product category | Yield (%) |
|---|---|
| Pyrolysis oil | 59.7 |
| Pyrolysis gas | 31.8 |
| Solid product | 8.5 |

**[Table 3]**

| | H₂ | CO₂ | CO | C1 | C2 | | C3 | | C4 |
|---|---|---|---|---|---|---|---|---|---|
| | | | | | paraffin | olefin | paraffin | olefin | |
| Composition (mol%) | 7.9 | 0.6 | 0.6 | 15.1 | 9.9 | 31.9 | 3.5 | 21.8 | 8.7 |

The pyrolysis gas was introduced into a wet scrubber containing an aqueous sodium hydroxide (NaOH) solution and purified to produce a first gas from which impurities were removed. The composition of impurities contained in the first gas was analyzed before and after introducing the first gas into the wet scrubber, and is shown in Table 4. As shown in Table 4, it was confirmed that impurities contained in the first gas were effectively removed through the purification process.

**[Table 4]**

| Impurity Gas | Before impurity removal (ppm) | After impurity removal (ppm) |
|---|---|---|
| HCl | 234 | <2 |
| H₂S | 1102 | <3 |
| COS | 19 | N/D |
| NH₃ | 337 | <2 |

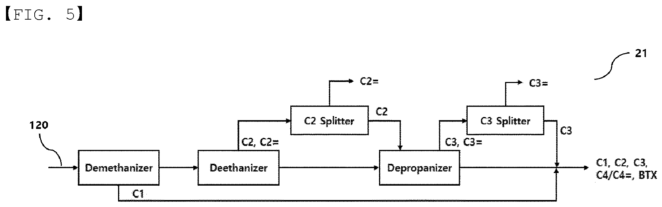

The first gas from which impurities were removed was passed through an olefin separation unit including a demethanizer, a deethanizer, and a depropanizer, as illustrated in FIG. 5, thereby separating olefins contained in the first gas. More specifically, the first gas was passed through the demethanizer to separate C1 compounds. The first gas from which the C1 compounds were separated was passed through the deethanizer to separate C2 compounds. The first gas from which the C2 compounds were separated was passed through the depropanizer to selectively separate only C3 compounds, thereby obtaining a purified first gas. The separated C2 compounds were separated into ethane (C₂H₆) and ethylene (C₂H₄) in a C2 splitter, and the separated C3 compounds were separated into propane (C₃H₈) and propylene (C₃H₆) in a C3 splitter. Ethylene and propylene were mixed with a finally produced light olefin stream, and C1 compounds, ethane, propane, butane, butylene, BTX (benzene, toluene, and xylene), and the like excluding light olefins were mixed to produce a purified first gas. The composition of the purified first gas obtained by passing through the olefin separation unit is shown in Table 5.

**[Table 5]**

| | Composition (mol%) | Weight (g) | Composition (mol) |
|---|---|---|---|
| H₂ | 16.9 | 15.5 | 7.8 |
| CO₂ | 1.3 | 25.5 | 0.6 |
| CO | 1.1 | 13.5 | 0.5 |
| C1 | 32.9 | 241.5 | 15.1 |
| C2 | 21.5 | 296.5 | 9.9 |
| C3 | 7.6 | 153.0 | 3.5 |
| C4 | 18.8 | 405.0 | 8.6 |
| total | 100 | 1150.5 | 46.0 |

100 mol of the purified first gas was introduced into a second reactor, which is a fluidized bed reactor packed with 100 g of an Ni/Al₂O₃ catalyst, and steam reforming was performed. The catalyst was used after being reduced under an H₂ gas atmosphere flowing at 900°C and a flow rate of 3.03 Nl/min. The steam reforming reaction was performed for 120 hours, and the steam reforming reaction was performed under conditions of a total flow rate of 3.33 Nl/min, a space velocity of 2.0 L/g_{cat}·h, and a steam-to-methane (S/C) ratio of 3. Thereafter, steam was removed to obtain a second gas. The compositions of the purified first gas and the second gas are shown in Table 6.

**[Table 6]**

| | Purified first gas (mol) | Second gas (mol) |
|---|---|---|
| Dry gas | 37.1 | 3.0 |
| CO₂ | 0.6 | 0.2 |
| CO | 0.5 | 73.4 |
| H₂ | 7.8 | 186.4 |

The second gas was introduced into an amine scrubber; a first stream containing carbon dioxide in the second gas was captured in the amine scrubber, and the second gas was separated into a second stream from which carbon dioxide was removed. Specifically, the second gas was introduced into a first amine scrubber; CO₂ was captured in an aqueous solution in which NH₃ was dissolved at 12 wt% under conditions of 50 to 60°C, 10 to 20 bar, a throughput of 0.5 Nl/hr, and a mol fraction of the second gas of 1, and uncaptured gas was recovered as a second stream. The solution used in the first amine scrubber was introduced into a second amine scrubber and separated into an aqueous NH₃ solution and CO₂ at 100°C, and CO₂ was recovered as a first stream.

The first stream was introduced into a fluidized bed reverse Boudouard reactor to produce a third stream through a reverse Boudouard reaction. In the reverse Boudouard reactor, the Ni/Al₂O₃ catalyst (coke ratio: 32.1%) that had been used in the steam reforming reaction was used, and high-purity graphite was additionally added to the reactor as an external carbon source. The reverse Boudouard reaction was performed under an N₂ gas atmosphere at 750°C, a CO₂ flow rate of 2.33 Nl/min, and a space velocity of 2.0 L/g_{cat}·h. The compositions of gases contained in the first stream, the second stream, and the third stream were analyzed and are shown in Table 7.

**[Table 7]**

| | First stream (mol) | Second stream (mol) | Third stream (mol) |
|---|---|---|---|
| Dry gas | 0 | 3.0 | N/D |
| CO₂ | 43.3 | 0.1 | 22.1 |
| CO | 0 | 73.1 | 40.7 |
| H₂ | 0 | 183.0 | N/D |
| Carbon | 21.3 | - | N/D |

The second stream and the third stream were mixed to produce a first syngas. The first syngas was converted into a second syngas having a CO:H₂ ratio of 1:2 through a water-gas shift reaction in the presence of a Cu/Zn/Al₂O₃ catalyst under reaction conditions of 165°C, 35 bar, and a space velocity of 1.4 L/g_{cat}·h. The second syngas was reintroduced into the amine scrubber to additionally remove residual carbon dioxide, and residual carbon dioxide separated from the first syngas was recovered to the reverse Boudouard reactor. The gas compositions of the first syngas, the second syngas, and the second syngas from which carbon dioxide was removed are shown in Table 8.

**[Table 8]**

| | First syngas (mol) | Second syngas (mol) | CO₂-removed second syngas (mol) |
|---|---|---|---|
| Dry gas | 3.0 | 2.8 | 2.7 |
| CO₂ | 22.2 | 37.1 | 1.5 |
| CO | 113.8 | 98.9 | 97.1 |
| H₂ | 183.0 | 197.9 | 191.1 |

The second syngas from which residual carbon dioxide was removed was introduced into a fixed bed reactor packed with a catalyst in which 30 wt% of Zr and 50 wt% of Cu were supported on Al₂O₃, thereby performing a methanol conversion reaction. In order to initiate reaction, 30 mol of CO₂ was additionally introduced into the reactor, and the methanol conversion reaction was performed under reaction conditions of 245°C, 35 bar, and 1.6 L/g_{cat}·h. The methanol conversion reaction was continuously recycled until the recovered methanol amount reached 2,750 g (recovery: 90 wt%), and a methanol-containing product produced after the reaction was recovered.

The recovered methanol was converted into light olefins in the presence of a ZSM-5 catalyst. A ZSM-5 catalyst was introduced into a fixed bed reactor, and light olefins were produced at 0.5 ml-MeOH/g_{cat}·h and 400°C. The produced light olefins were mixed with ethylene and propylene separated in the olefin separation unit, and an olefin product was recovered. Thereafter, moisture was removed to recover olefins only.

The gas composition analysis was performed by gas chromatography (GC), and the total amount of gas was confirmed using a gas meter. Specifically, selectivity for each gas was calculated based on quantification by GC, and the composition of each gas was analyzed based on the total gas amount confirmed by the gas meter. In this case, the term "dry gas" collectively refers to hydrocarbon gases having 4 or fewer carbon atoms.

Referring to Table 6, it can be seen that dry gas and carbon dioxide were consumed through the reforming reaction of the first gas and were converted into carbon monoxide and hydrogen. Table 7 shows that the second gas produced through the steam reforming reaction was separated, by an amine scrubber, into a second stream from which residual CO₂ contained in the second gas was removed and a first stream containing residual CO₂, and that the first stream was subjected to a reverse Boudouard reaction to convert carbon dioxide into carbon monoxide, thereby producing a third stream. In detail, the first stream contained 43.3 mol of carbon dioxide and did not contain carbon monoxide. However, as carbon dioxide contained in the first stream was converted into carbon monoxide through a reverse Boudouard reaction, it was confirmed that the third stream contained 22.1 mol of carbon dioxide and 40.7 mol of carbon monoxide. Accordingly, it can be seen that carbon dioxide was converted into carbon monoxide by the reverse Boudouard reaction.

According to the present disclosure, by recovering carbon dioxide contained in the second gas and then subjecting the recovered carbon dioxide to a reverse Boudouard reaction to produce syngas, the amount of syngas obtained may be increased, the content of carbon dioxide in the syngas may be reduced, and, at the same time, the syngas may contain a larger amount of carbon monoxide and hydrogen. Accordingly, the yield of light olefins finally produced may be improved.

In particular, as the steam reforming reaction is performed, catalyst deactivation due to coke formed during the reaction may be minimized, thereby achieving a high syngas production yield. Further, even when coke is deposited on the catalyst during the steam reforming reaction and the catalyst is deactivated, the deactivated catalyst may be introduced into the reverse Boudouard reaction so that the coke on the catalyst surface is used as a carbon source for the reverse Boudouard reaction. Accordingly, the catalyst may be easily regenerated only by performing the reverse Boudouard reaction, thereby enabling a continuous process.

Referring to Table 8, it can be seen that, after the reverse Boudouard reaction process, a second syngas in which the ratio of carbon monoxide to hydrogen was controlled to about 1:2 was produced through a water-gas shift process. By controlling the ratio of carbon monoxide to hydrogen contained in the syngas, a subsequent olefin conversion reaction may be efficiently performed, thereby significantly improving the yield of hydrocarbons obtained. In detail, the second syngas subjected to the water-gas shift process contained 197.9 mol of H₂ and 98.9 mol of CO, whereas the second syngas contained a small amount of CO₂, 37.1 mol, thereby providing advantageous effects in terms of improving a syngas production yield and preventing environmental pollution.

As the syngas production yield was improved, after the syngas was converted into methanol, the yield of light olefins produced from the methanol was increased. Specifically, when light olefins were produced by the method of Example 1, the methanol conversion was 99.3%, and the olefin selectivity was 81%, indicating a significant improvement in olefin production yield. At the same time, the selectivity for C4 or higher hydrocarbons was as low as 11%, and among the total olefins recovered, the selectivities for ethylene and propylene were measured to be 5.1% and 41.2%, respectively, confirming that the selectivity for light olefins was significantly improved. In particular, by separating ethylene and propylene contained in the first gas in the ethylene separation unit and mixing the separated ethylene and propylene with the light olefin product, the yield of light olefins was improved, and the process was operated more efficiently.

Accordingly, when light olefins are produced by the method according to the present disclosure, process efficiency is improved, thereby enabling syngas to be produced at a high yield and improving the yield of light olefins produced therefrom. In detail, by performing the steam reforming process and the reverse Boudouard reaction process, irreversible deactivation of the catalyst may be minimized, thereby enabling a continuous process. By recovering carbon dioxide in the mixed gas and converting the recovered carbon dioxide into carbon monoxide, the syngas production yield is improved, and greenhouse gas emissions are reduced. In addition, since syngas in which the ratio of carbon monoxide to hydrogen in the mixed gas is controlled is produced through a water-gas shift process, a subsequent process is performed more efficiently, thereby significantly improving the production yield of light olefins.

Although the present invention has been described with reference to specific matters and limited embodiments, such description has been provided only to assist in a general understanding of the present invention. Therefore, the present invention is not limited to the embodiments, and various modifications and changes may be made by those skilled in the art to which the present invention pertains from this description.

Therefore, the spirit of the present invention should not be construed as being limited to the described embodiments, and not only the claims set forth below but also all equivalents and modifications thereof are intended to fall within the scope of the present disclosure.

**[Detailed Description of Main Elements]**

| | | | |
|---|---|---|---|
| 10: | First reactor | 20: | Purification unit |
| 21: | Olefin separation unit | 30: | Second reactor |
| 40: | First syngas production unit | 41: | Amine scrubber |
| 42: | Reverse Boudouard reactor | 50: | Second syngas production unit |
| 60: | Third reactor | 70: | Fourth reactor |
| 100: | Waste plastic pyrolysis product | 110: | Pyrolysis gas |
| 120: | First gas | 130: | Second gas |
| 140: | First syngas | 150: | Second syngas |
| 160: | Methanol-containing product | 170: | Olefin-containing product |
| 210: | First stream | 220: | Second stream |
| 230: | Third stream | 240: | Olefin product |
| 300: | Waste plastics | 310: | Pyrolysis oil |

## Claims

1. A method for producing light olefins, the method comprising:
S1) pyrolyzing waste plastics to produce pyrolysis oil and pyrolysis gas;
S2) separating the pyrolysis oil and the pyrolysis gas;
S3) purifying the separated pyrolysis gas to produce a first gas from which impurities are removed;
S4) subjecting the first gas to a steam reforming reaction to produce a second gas;
S5) producing a first syngas from the second gas;
S6) converting carbon monoxide in the first syngas into hydrogen and carbon dioxide through a water-gas shift reaction to produce a second syngas;
S7) converting the second syngas into methanol through a methanol conversion reaction; and
S8) converting the methanol into light olefins through an olefin conversion reaction.

2. The method of claim 1, wherein the step S3) includes:
removing impurities from the separated pyrolysis gas to produce the first gas; and
separating olefins from the first gas.

3. The method of claim 2, wherein the olefins are mixed with the light olefins in the step S8) and recovered.

4. The method of claim 2, wherein the impurities include one or two or more selected from the group consisting of tar, sulfur, nitrogen, and chlorine.

5. The method of claim 1, wherein, in the step S4), the second gas contains hydrogen, carbon monoxide, and carbon dioxide.

6. The method of claim 1, wherein the step S5) includes:
separating, from the second gas, a first stream containing carbon dioxide and a second stream containing hydrogen and carbon monoxide;
converting the first stream into a third stream containing carbon monoxide through a reverse Boudouard reaction; and
mixing the third stream and the second stream to produce the first syngas.

7. The method of claim 1, wherein the pyrolysis oil contains one or more fractions selected from the group consisting of an aromatic fraction, naphtha, and a heavy fraction.

8. The method of claim 1, wherein a pyrolysis temperature in the step S1) is 400°C to 600°C.

9. The method of claim 1, wherein, in the step S4), the steam reforming reaction is performed in the presence of a catalyst, and the catalyst is a composite catalyst in which a hydrogenation metal is supported on a support.

10. The method of claim 9, wherein the hydrogenation metal is one or more selected from the group consisting of nickel, vanadium, iron, platinum, palladium, and ruthenium.

11. The method of claim 6, wherein the reverse Boudouard reaction is performed at a temperature of 800°C to 1,000°C and a pressure of 50 KPa to 200 KPa.

12. The method of claim 1, wherein the second syngas contains hydrogen and carbon monoxide, and a molar ratio of hydrogen to carbon monoxide is 1.9 to 2.1:1.

13. The method of claim 1, wherein the step S7) is performed at a temperature of 400°C to 600°C and a pressure of 1 bar to 10 bar.

14. The method of claim 1, wherein the methanol conversion product in the step S7) contains methanol in an amount of 10 wt% or more based on the total weight of the product.

15. The method of claim 1, wherein the step S8) is performed in the presence of a zeolite-based catalyst or an AlPO₄-based molecular sieve catalyst.

16. The method of claim 15, wherein the zeolite-based catalyst or the AlPO₄-based molecular sieve catalyst is ZSM-5, SAPO-34, or a combination thereof.

17. An apparatus for producing light olefins, the apparatus comprising:
a first reactor configured to perform pyrolysis of organic waste to produce pyrolysis oil and pyrolysis gas;
a purification unit configured to receive the pyrolysis gas and produce purified pyrolysis gas;
a second reactor configured to receive the purified pyrolysis gas and produce a second gas through a steam reforming reaction;
a first syngas production unit configured to produce a first syngas from the second gas;
a second syngas production unit configured to convert carbon monoxide in the first syngas into hydrogen and carbon dioxide through a water-gas shift reaction to produce a second syngas;
a third reactor configured to produce methanol from the second syngas through a methanol conversion reaction; and
a fourth reactor configured to produce light olefins from the methanol through an olefin conversion reaction.

18. The apparatus of claim 17, wherein the purification unit includes: an impurity removal unit configured to remove impurities; and an olefin separation unit configured to separate and recover olefins.

19. The apparatus of claim 17, wherein the first syngas production unit includes: an amine scrubber configured to receive the second gas and separate carbon dioxide; and a reverse Boudouard reactor configured to receive the carbon dioxide separated from the amine scrubber and perform a reverse Boudouard reaction.

20. The apparatus of claim 17, wherein the first to fourth reactors include a fluidized bed reactor or a fixed bed reactor.

21. The apparatus of claim 19, wherein the reverse Boudouard reactor includes a fluidized bed reactor or a fixed bed reactor.
